# EUROPEAN PATENT SPECIFICATION

(11) **EP 2 020 230 B1**
(45) Date of publication and mention of the grant of the patent: **19.01.2011**
(21) Application number: 07384032.4
(22) Date of filing: 01.08.2007
(51) Int. Cl.: A61K 31/192, A61K 31/4045, A61K 31/4155, A61K 31/428, A61K 31/429, A61K 31/433, A61K 31/4439, A61K 31/454, A61K 31/4709, A61K 31/496, A61K 31/506, A61K 31/5377, A61K 31/63, A61P 25/00

(54) **Combination of at least two 5-HT6-Ligands**
Kombination von mindestens zwei 5-HT6-Liganden
Combinaison d'au moins deux ligands 5-HT6

(43) Date of publication of application: 04.02.2009
(73) Proprietor: Laboratorios del Dr. Esteve S.A., 08041 Barcelona (ES)
(72) Inventor: Codony-Soler, Xavier, 08301 Mataro (Barcelona) (ES); Vela-Hernandez, José-Miguel, 08028 (Barcelona) (ES); Buschmann, Helmut H., Dr., 08960 Sant Just Desvern (Barcelona) (ES)
(74) Representative: Peters, Hajo

(56) References cited:
- EP-A- 1 632 491
- EP-A- 1 676 841
- EP-A- 1 747 779
- WO-A-02/08178
- WO-A-2005/013974
- WO-A-2005/013976
- WO-A-2005/013977
- WO-A-2005/013978
- WO-A-2005/013979
- WO-A-2006/015867
- WO-A-2006/037482
- WO-A-2007/054257
- US-A1- 2003 191 124
- SVENNINGSSON, PER ET AL: "Biochemical and behavioral evidence for antidepressant-like effects of 5-HT6 receptor stimulation" THE JOURNAL OF NEUROSCIENCE, vol. 27, no. 15, 11 April 2007 (2007-04-11), pages 4201-4209, XP002459783 ISSN: 0270-6474
- DE FOUBERT ET AL: "Acute onset by 5-HT6-receptor activation on rat brain brain-derived neurotrophic factor and activity-regulated cytoskeletal-associated protein mRNA expression" NEUROSCIENCE, NEW YORK, NY, US, vol. 147, no. 3, 29 June 2007 (2007-06-29), pages 778-785, XP022134973 ISSN: 0306-4522
- ROTH B L ET AL: "Serotonin receptors represents highly favorable molecular targets for cognitive enhancement in schizophrenia and other disorders" PSYCHOPHARMACOLOGY, SPRINGER VERLAG, BERLIN, DE, vol. 174, no. 1, 2004, pages 17-24, XP002441201 ISSN: 0033-3158
- "P.1.045 Binding of 3H-WIN-35,428 and 125I-RTI-121 to human platelet membranes" EUROPEAN NEUROPSYCHOPHARMACOLOGY, ELSEVIER SCIENCE PUBLISHERS BV, AMSTERDAM, NL, vol. 15, 2005, page S382, XP005556611 ISSN: 0924-977X
- "P.1.044 WAY-181187: neurochemical profile of a novel and selective 5-HT6 receptor agonist" EUROPEAN NEUROPSYCHOPHARMACOLOGY, ELSEVIER SCIENCE PUBLISHERS BV, AMSTERDAM, NL, vol. 15, 2005, page S382, XP005556610 ISSN: 0924-977X
- 'Add-On Therapy Improves Depressive Symptoms In Bipolar Disorder' SCIENCEDAILY, [Online] Elsevier Retrieved from the Internet: <URL:http://www.sciencedaily.com/releases/2 008/09/080902075218.htm> [retrieved on 2008-09-07]
- 'Small Trial Shows Daclizumab Add-On Therapy Improves Multiple Sclerosis Outcome', [Online] National Institute of Neurological Disorders and Stroke Retrieved from the Internet: <URL:http://www.ninds.nih.gov/news_and_even ts/press_releases/pressrelease_ms_daclizuma b_052404.htm> [retrieved on 2004-05-24]
- FONE K.C.F.: 'Selective 5-HT6 compounds as a novel approach to the treatment of Alzheimer's disease' JOURNAL OF PHARMACOLOGICAL SCIENCES, JAPANESE PHARMACOLOGICAL SOCIETY, TOKYO, JP vol. 101, 01 January 2006, page 53, XP009111264 ISSN: 1347-8613
- HEAL D.J. ET AL: 'Selective 5-HT6 receptor ligands: Progress in the development of a novel pharmacological approach to the treatment of obesity and related metabolic disorders' PHARMACOLOGY AND THERAPEUTICS vol. 117, no. 2, 30 October 2007, ELSEVIER, GB, pages 207 - 231, XP022432141 ISSN: 0163-7258
- MENESES ET AL: "The effects of the 5-HT6 receptor agonist EMD and the 5-HT7 receptor agonist AS19 on memory formation" BEHAVIOURAL BRAIN RESEARCH, vol. 195, 11 January 2008 (2008-01-11), pages 112-119,
- PAUWELS PETRUS ET AL: 'Memory enhancing properties of E-6801, a 5-HT6 receptor ligand with agonist properties, in the novel object discrimination test' ACTA PHARMACOLOGICA SINICA vol. 27, no. SUPPL.1, 01 July 2006, WILEY INTERSCIENCE, page 76, XP009119099 ISSN: 1671-4083

## Description

The present invention relates to a Combination of at least two 5HT6- Ligands of which one is a partial or full agonist while the other is a full antagonist or an inverse agonist, a medicament comprising this comination, the use of the combination in the manufacture of a medicament for the treatment of cognintive or degenerative brain disorders, memory disorders, or ADHD, or for memory enhancement, and methods of treatment using the combination or its respective members in a dosing pattern.

Cognitive and/or degenerative brain disorders are characterized clinically by progressive loss of memory, cognition, reasoning, judgement and emotional stability that gradually leads to profound mental deterioration and ultimately death. In an example of such disorders, Alzheimer's disease is a common cause of progressive mental failure (dementia) in aged humans and is believed to represent the fourth most common medical cause of death in the United States. In particular, Alzheimer's disease is associated with degeneration of cholinergic neurons in the basal forebrain that play a fundamental role in cognitive functions, including memory. Cognitive and/or degenerative brain disorders have been observed in varied races and ethnic groups world-wide and presents a major public health problem. These diseases are currently estimated to affect about two to three million individuals in the United States alone and the occurrence will increase world-wide as the human life span increases. On the other hand shortcomings or failure to use the memory to its full abilities is a common problem and thus also often needs pharmaceutical attention

In particular, it was an object of the present invention to provide a medicament suitable for the prophylaxis and/or treatment of cognitive disorders or for memory enhancement.

Said object has been achieved by providing a combination of active substances comprising at least one compound (A)
being selected from compounds binding to the 5HT6-receptor and acting as an full agonist or partial agonist, said compound being N-[3-(2-dimethylamino-ethyl)-1*H*-indol-5-yl]-6-chloroimidazo[2,1-b]thiazol-5-sulphonamide
   and at least one compound (B)
being selected from compounds binding to the 5HT6-receptor and acting as an antagonist or inverse agonist, said compound being SB-271046.

It has now been surprisingly demonstrated that an active substance combination comprising a 5HT6 ligand being a full or partial agonist being N-[3-(2-dimethylamino-ethyl)-1*H*-indol-5-yl]-6-chloroimidazo[2,1-b]thiazol-5-sulphonamide and a 5HT6 ligand being a full antagonist or inverse agonist being SB-271046 was able to positively act on the CNS activities in a mammal by acting in models of cognitive disorders especially by enhancing the memory of a mammal.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is a serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther., 1995, 274, 173; A.J. Sleight, et al., Behav. Brain Res., 1996, 73, 245; T.A. Branchek, et al., nnu. Rev. Pharmacol. Toxicol., 2000, 40, 319; C. Routledge, et al., Br. J. Pharmacol., 2000, 130, 1606]. It has been shown that typical and atypical antipsychotic drugs for treating schizophrenia have a high affinity for 5-HT₆ receptors [B.L. Roth, et al., J. Pharmacol. Exp. Ther., 1994, 268, 1403; C.E. Glatt, et al., Mol. Med., 1995, 1, 398; F.J. Mosma, et al., Mol. Pharmacol., 1993, 43, 320; T. Shinkai, et al., Am. J. Med. Genet., 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol., 2000, 130, 1597; C. Gérard, et al., Brain Research, 1997, 746, 207; M.R. Pranzatelli, Drugs of Today, 1997, 33, 379]. Moreover, it has been shown that the 5-HT₆ receptor also plays a role in food ingestion [Neuropharmacology, 41, 2001, 210-219].

"Compound/s binding to the 5HT6-receptor" (with "5HT6-Ligand" being also used in this description and being one and the same as "Compound/s binding to the 5HT6-receptor") as used in this application is/are defined as having on the 5HT6-receptor an K_{¡} value of ≤ 5000 nM. More preferably the "compound/s binding to the 5HT6-receptor" are having on the 5HT6-receptor an Kᵢ value of ≤ 1000 nM, more preferably ≤ 500 nM. More preferably, the Kᵢ value its ≤ 250 nM. More preferably, the Kᵢ value is ≤ 100 nM.More preferably, the Kᵢ value is ≤ 100 nM. Most preferably, the Kᵢ value is ≤ 50 nM. An also fitting definition is to define the compounds by way of their IC₅₀ values and thus "compound/s binding to the 5HT6-receptor" as used in this application is also understood as meaning compounds having on the 5HT6-receptor an IC₅₀ value of ≤ 5000 nM. More preferably the "compound/s binding to the 5HT6-receptor" are having on the 5HT6-receptor an IC₅₀ value of ≤ 1000 nM, more preferably of ≤ 500 nM. More preferably, the IC₅₀ value is ≤ 250 nM. More preferably, the IC₅₀ value is ≤ 100 nM. Most preferably, the IC₅₀ value is ≤ 50 nM. Compound binding to the 5HT6-receptor may be partial agonists, antagonists, full agonists, or inverse agonists. Pharmacological test systems to determine all of these functionalities are well-known in the art.

The active substance combination according to this invention comprises preferably 1-99% by weight of the component (A) N-[3-(2-dimethylamino-ethyl)-1*H*-indol-5-yl]-6-chloroimidazo[2,1-b]thiazol-5-sulphonamide and 99-1 % by weight of the component (B), SB-271046, more preferably 10-80% by weight of the component (A) N-[3-(2-dimethylamino-ethyl)-1*H*-indol-5-yl]-6-chloroimidazo[2,1-b]thiazol-5-sulphonamide and 80-20% by weight of the component (B) SB-271046, these percentages referring to the total weight of both components (A) and (B).

Assays that may be used for determining the affinity and selectivity of a 5-HT7 receptor agonist and/or other affinities to 5-HT receptors are well known in the art and especially measuring the affinities to these receptors are offered by service companies like. It is possible to classify a compound with 5-HT receptor affinity as full or partial agonist (also as inverse agonist or antagonist) according to the reference of S. M. Stahl, Essential Psychopharmacology, Neuroscientific basis and practical applications, Ed. Cambridge, 1996, Chapter 3. The respective part of the literature is hereby incorporated by reference and forms part of the disclosure.

As defined herein it is prefered if the functionality of the compounds (A) N-[3-(2-dimethylamino-ethyl)-1*H*-indol-5-yl]-6-chloroimidazo[2,1-b]thiazol-5-sulphonamide and (B) SB-271046 both binding to the 5HT6 receptor, the 5HT6 ligand, is determined in regards to the same biological group of mammals. Thus, to select and define a Compound (A) N-[3-(2-dimethylamino-ethyl)-1*H*-indol-5-yl]-6-chloroimidazo[2,1-b]thiazol-5-sulphonamide as being a 5HT6 ligand and acting as a partial or full agonist, and to select and define a Compound (B) SB-271046 as being a 5HT6 ligand and acting as an antagonist or inverse agonist, the same test systems, especially in regards to the species being used, should be employed. So, to determine and define the affinity and functionality of Compound (A) N-[3-(2-dimethylamino-ethyl)-1*H*-indol-5-yl]-6-chloroimidazo[2,1-b]thiazol-5-sulphonamide and Compound (B) SB-271046 to be used in the same combination of active substances according to the invention the animals or organs (if non-human), cells, cell systems, nucleic acids, receptors, proteins or peptides used to determine affinity and functionality should be from the same species, e.g. rat, mouse, human.

Especially preferred embodiments of the invention encompass the use of a compound with a very specific binding to the 5HT6 receptor being in its binding profile more specific in its affinity (thus showing a lower Ki) to the 5HT6 receptor than in its affinity to other 5HT Receptors said compounds being N-[3-(2-dimethylamino-ethyl)-1*H*-indol-5-yl]-6-chloroimidazo[2,1-b]thiazol-5-sulphonamide and SB-271046.

Therefore in a preferred embodiment of the invention the compounds (A) N-[3-(2-dimethylamino-ethyl)-1*H*-indol-5-yl]-6-chloroimidazo[2,1-b]thiazol-5-sulphonamide and (B) SB-271046, preferably both are binding to the 5HT6 receptor with a higher affinity - expressed as a lower Ki-value - to the 5HT6 receptor than to the 5-HT1A or 5HT7 receptor; especially binding with an affinity higher by a factor of at least 10, preferably with an affinity higher by a factor of at least 30, more preferably with an affinity higher by a factor of at least 50, most preferably with an affinity higher by a factor of at least 100.

By way of exemplifying the above paragraph a compound X - specific to the 5HT6 receptor -, is assumed to have an affinity - expressed as a Ki value - of 1 nM and an affinity to the 5HT1A receptor of a Ki value of 42 nM and thus would have an affinity to the 5HT6 receptor higher (over the 5HT1A receptor) by a factor of 42.

In another preferred embodiment of the invention the compounds (A) N-[3-(2-dimethylamino-ethyl)-1*H-*indol-5-yl]-6-chloroimidazo[2,1-b]thiazol-5-sulphonamide and (B) SB-271046, preferably both are binding to the 5HT6 receptor with a Kᵢ value of ≤ 5000 nM, preferably of ≤ 1000 nM, more preferably of ≤ 500 nM. Preferably, the Kᵢ value is ≤ 250 nM, or ≤ 100 nM, or most preferably is ≤ 50 nM.

In another preferred embodiment of the invention the compounds (A) N-[3-(2-dimethylamino-ethyl)-1*H*-indol-5-yl]-6-chloroimidazo[2,1-b]thiazol-5-sulphonamide and/or (B) SB-271046, preferably both are binding to the 5HT6 receptor with a higher affinity to the 5HT6 receptor than to the 5-HT1A or 5HT7 receptor; especially binding with an affinity higher by a factor of at least 10, preferably with an affinity higher by a factor of at least 30, more preferably with an affinity higher by a factor of at least 50, most preferably with an affinity higher by a factor of at least 100;
and
the compounds (A) N-[3-(2-dimethylamino-ethyl)-1*H*-indol-5-yl]-6-chloroimidazo[2,1-b]thiazol-5-sulphonamide and (B) SB-271046, preferably both are binding to the 5HT6 receptor with a Kᵢ value of ≤ 5000 nM, preferably of ≤ 1000 nM, more preferably of ≤ 500 nM. Preferably, the Kᵢ value is ≤ 250 nM, or ≤ 100 nM, or most preferably its ≤ 50 nM.

An "agonist" is defined as a compound that binds to a receptor and has an intrinsic effect, and thus, increases the basal activity of a receptor when it contacts the receptor. Full agonists show the maximum effect on the 5-HT6 receptor, whereas a partial agonist is giving less (e.g. 80%) of the response of the full agonist as a maximum.

An "antagonist" is defined as a compound that competes with an agonist or inverse agonist for binding to a receptor, thereby blocking the action of an agonist or inverse agonist on the receptor. However, an antagonist (also known as a "neutral" antagonist) has no effect on constitutive receptor activity.

An "inverse agonist" is defined as a compound that produces an effect opposite to that of the agonist by occupying the same receptor and, thus, decreases the basal activity of a receptor (i.e., signalling mediated by the receptor). Such compounds are also known as negative antagonists. An inverse agonist is a ligand for a receptor that causes the receptor to adopt an inactive state relative to a basal state occurring in the absence of any ligand. Thus, while an antagonist (or neutral antagonist) can inhibit the activity of an agonist, an inverse agonist is a ligand that can alter the conformation of the receptor in the absence of an agonist. SB-271046 (5-Chloro-N-(4-methoxy-3-(piperazin-1-yl)phenyl)-3-methyl-2-benzothiophene sulfonamide) is described in Bromidge et al., J.Med.Chem 48, 353-356 (1999), Thus, a further aspect of the invention is a medicament comprising a combination of active substances according to the invention and optionally at least one pharmaceutical adjuvant.

In one preferred embodiment the medicament comprising a combination of active substances comprises at least one compound (A), wherein the at least one compound (A) is N-[3-(2-dimethylamino-ethyl)-1*H*-indol-5-yl]-6-chloroimidazo[2,1-b]thiazol-5-sulphonamide and at least one compound (B), wherein the at least one compound (B) is SB-271046, and wherein the medicament optionally also comprises at least one pharmaceutical adjuvant.

Another further aspect of the invention thus is also a pharmaceutical formulation comprising a combination of active substances according to the invention and optionally at least one pharmaceutical adjuvant. This pharmaceutical formulation may also be formulated into a medicament.

In a preferred embodiment of the medicament according to the invention the medicament is for the treatment of Peripheral Nervous System Disorders, or Central Nervous System Disorders, especially Central Nervous System Disorders.

In another preferred embodiment of the medicament according to the invention the medicament is for the treatment of cognitive disorders, memory disorders, senile dementia processes, such as Alzheimer's, Parkinson's and/or Huntington's Disease, attention deficit disorder, such as infantile hyperkinesia (ADHD, attention deficit / hyperactivity disorder), epilepsy, anxiety, panic, depression, psychosis, pain, schizophrenia; or for the improvement/enhancement of cognition.

In a preferred embodiment of the Medicament according to the invention the medicament is for the treatment of cognitive disorders, degenerative disorders, memory disorders, ADHD (attention deficit / hyperactivity disorder), Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder, infantile hyperkinesia (ADHD, attention deficit/hyperactivity disorder); especially ADHD, or for the improvement/enhancement of cognition.

Thus, a further aspect of the invention is the use of a combination of active substances according to the invention for the manufacture of a medicament for the treatment of Peripheral Nervous System Disorders, or Central Nervous System Disorders, especially Central Nervous System Disorders.

"Treatment" as used in this application is defined as the treatment of a disease or of a medically relevant symptom, but also includes the prevention of the symptom or disease preventive/prophylactic activity during or before the development of the symptom or disease.

In a preferred embodiment of the use according to invention the medicament manufactured is for the treatment of cognitive disorders, memory disorders, senile dementia processes, such as Alzheimer's, Parkinson's and/or Huntington's Disease, attention deficit disorder, such as infantile hyperkinesia (ADHD, attention deficit / hyperactivity disorder), epilepsy, anxiety, panic, depression, psychosis, pain, schizophrenia; or for the improvement/enhancement of cognition.

In another preferred embodiment of the use according to invention the medicament manufactured is for the treatment of cognitive disorders, degenerative disorders, memory disorders, ADHD (attention deficit / hyperactivity disorder), Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder, infantile hyperkinesia (ADHD, attention deficit / hyperactivity disorder); especially ADHD, or for the improvement/enhancement of cognition.

The medicament may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible pharmaceutical adjuvants like carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain other pharmaceutical adjuvants like additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions (or medicaments) of the present invention may also be administered topically or via a suppository.

The above mentioned compositions (or medicaments) include preferably 1 to 60 % by weight of the combination of active substances according to the invention, and 40 to 99 % by weight of the appropriate pharmaceutical vehicle(s).

The daily dosage applied to a patient/mammal of the combination of active substances according to the invention and also of each of its respective Compounds (A) N-[3-(2-dimethylamino-ethyl)-1*H*-indol-5-yl]-6-chloroimidazo[2,1-b]thiazol-5-sulphonamide or (B) SB-271046 being 5HT6 ligands may vary depending on factors that have their basis in the respective species or other factors, such as age, weight or degree of illness and so forth. The daily dosage for mammals including humans of the combination of active substances according to the invention and also of each of its respective Compounds (A) N-[3-(2-dimethylamino-ethyl)-1*H*-indol-5-yl]-6-chloroimidazo[2,1-b]thiazol-5-sulphonamide or (B) SB-271046usally ranges from 1 milligram to 2000 milligram, preferably 1 to 1500 mg, more preferably 1 to 1000 mg of substance to be administered during one or several intakes.

As a further aspect the invention also provides a method of treatment for cognitive disorders, memory disorders or degenerative brain disorders by applying to a mammal or patient in need thereof a suitable amount of a 5HT6 ligand acting as a partial or full agonist being N-[3-(2-dimethylamino-ethyl)-1*H*-indol-5-yl]-6-chloroimidazo[2,1-b]thiazol-5-sulphonamide and of a 5HT6 ligand acting as a full antagonist or inverse agonist being SB-271046 in the form of a combination of active substances according to the invention. Or, in a further embodiment the 5HT6 ligand acting as a partial or full agonist being N-[3-(2-dimethylamino-ethyl)-1*H*-indol-5-yl]-6-chloroimidazo[2,1-b]thiazol-5-sulphonamide is applied at the same time when and the 5HT6 ligand acting as a full antagonist or inverse agonist being SB-271046 is applied, whereas this may be achieved through the same pharmaceutical pathway or even the same medicament (as a combination of the active substances according to the invention).

The following figures and examples are provided to illustrate the claimed invention .

### Figures:

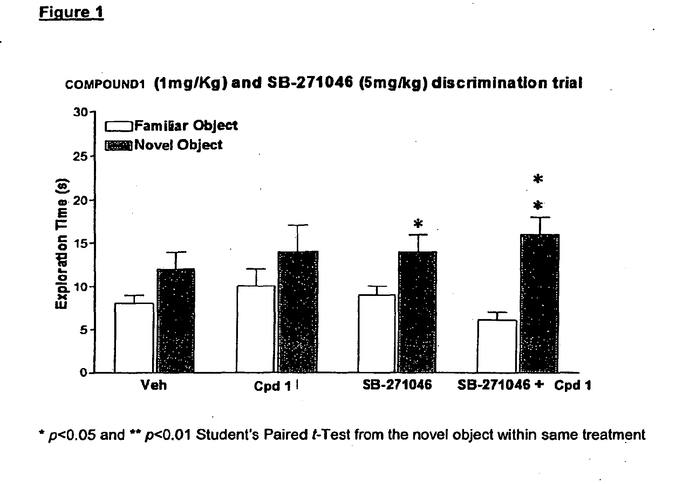

- Figure 1:: Effect of co-administration of COMPOUND 1, a 5HT6 ligand acting as agonist on the 5HT6 receptor and the 5HT6 ligand SB-271046 acting as antagonist on the 5HT6 receptor. As can be clearly seen the combined treatmen of COMPOUND 1 and SB-271046 did result in a quite pronounced effect, even an synergistic effect, over the use of each of the substances alone, which at these doses were either not or very low effective in other dose response studies (see below). * *p*<0.05 and ** *p*<0.01 Student's Paired *t*-Test from the novel object within same treatment.

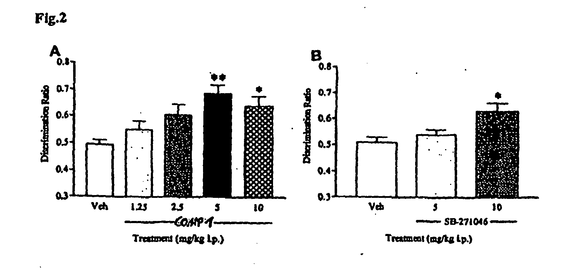

- Figure 2:: Dose response trials using either A) COMPOUND 1 (at 1.25, 2.5, 5 or 10 mg/kg i.p.) or B) SB-271046 (at 5 or 10 mg/kg i.p.) the maximum effect was achieved with COMPOUND 1 at 5 mg/kg and for SB-271046 at 10 mg/kg, with neither COMPOUND 1 at 1 mg/kg nor SB-271046 at 5 mg/kg being significantly effective. * *p*<0.05 and ** *p*<0.01 Student's Paired *t*-Test from the novel object within same treatment.

### Examples:

For exemplifying the of the combibination SB-271046 was used. SB-271046 is a well-known compound binding to the 5HT6 receptor and acting as an antagonist.

The other compound used was 6-Chloro-imidazo[2,1-b]thiazole-5-sulfonic acid [3-(2-dimethylamino-ethyl)-1H-indol-5-yl]-amide (hereinafter called COMPOUND 1):

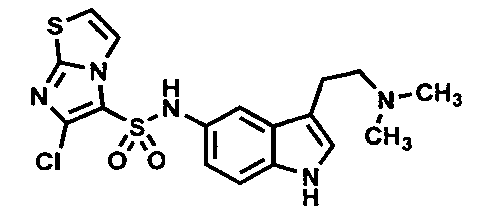

This compound is known from WO 03/42175 A1 and is an agonist with a very low Kᵢ in binding to the 5HT6 receptor.

### Example 1 (Novel object discrimination trial):

### Methods

Adult male Lister Hooded rats (Charles River, UK) weighing 200-350g at the start of the experiment were housed in groups of four on a 12:12 h light:dark cycle (lights on at 07:00 h). Food and water were available *ad libitum* throughout the study, and the room temperature (21 ± 2°C) and relative humidity (45-65%) were kept constant. A group of rats (n=12 each) received injection of a sub-effective dose of COMPOUND 1 (1mg/kg i.p.) or vehicle (0.5% methylcellulose in saline, 2ml/kg), either alone or combined with SB-271046 (5mg/kg). Each drug combination was administered to all rats in the group over a period of 4 weeks in a random order using a seven day behavioural test interval.

The two trial novel object discrimination paradigm utilised, was as described by Ennaceur and Delacour, (1988) with minor modification (King *et al*., 2004b; Woolley *et al*., 2003). The twelve open field test arenas used for object discrimination were clear perspex boxes (39 × 23.5 cm with 24.5 cm high walls) to which each rat was habituated for 60 minutes the day prior to test days. On the test day the first drug was administered -40 minutes and the second drug -20 minutes before the familiarisation trial. Therefore, 20 minutes after the injection each rat received 3 minutes acclimatisation to the perspex box in absence of objects which was then followed by the 3 minute familiarisation trial and a second 3 minute choice trial following a 4 hour inter-trial interval. During both trials, exploration of each object was defined as the time spent (s) sniffing (within 1 cm of it with active vibrissae), licking, chewing or touching the object with the nose.

As can be clearly seen in Figure 1 the combined treatment of COMPOUND 1 and SB-271046 did result in a quite pronounced effect, even an synergistic effect, over the use of each of the substances alone.

In previous dose response trials according to above description (on the test day the respective drug was administered -20 minutes before the familiarisation trial) using either COMPOUND 1 (at 1.25, 2.5, 5 or 10 mg/kg i.p.) or SB-271046 (at 5 or 10 mg/kg i.p.) the maximum effect was achieved with COMPOUND 1 at 5 mg/kg and for SB-271046 at 10 mg/kg, with neither COMPOUND 1 at 1mg/kg nor SB-271046 at 5 mg/kg being significantly effective (See Fig. 2 A) and B)).

## Claims

1. A medicament comprising a combination of active substances comprising at least one compound (A), wherein the at least one compound (A) is N-[3-(2-dimethylamino-ethyl)-1*H*-indol-5-yl]-6-chloroimidazo[2,1-b]thiazol-5-sulphonamide
and at least one compound (B), wherein the at least one compound (B) is SB-271046, and wherein the medicament optionally also comprises at least one pharmaceutical adjuvant.

2. Medicament according to claim 1 for the treatment of Peripheral Nervous System Disorders, or Central Nervous System Disorders, especially Central Nervous System Disorders.

3. Medicament according to claim 1 or 2 for the treatment of cognitive disorders, memory disorders, senile dementia processes, such as Alzheimer's, Parkinson's and/or Huntington's Disease, attention deficit disorder, such as infantile hyperkinesia (ADHD, attention deficit / hyperactivity disorder), epilepsy, anxiety, panic, depression, psychosis, pain, schizophrenia; or for the improvement/enhancement of cognition.

4. Medicament according to any of claims 1 to 3 for the treatment of cognitive disorders, degenerative disorders, memory disorders, ADHD (attention deficit / hyperactivity disorder), Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder, infantile hyperkinesia (ADHD, attention deficit/hyperactivity disorder); especially ADHD, or for the improvement/enhancement of cognition.

## Patentansprüche

1. Medikament, das eine Kombination von Wirksubstanzen umfasst, welche mindestens eine Verbindung (A) umfassen, wobei die mindestens eine Verbindung (A) N-[3-(2-Dimethylamino-ethyl)-1*H*-indol-5-yl]-6-chloroimidazo[2,1-b]thiazol-5-sulfonamid ist, und mindestens eine Verbindung (B) umfassen, wobei die mindestens eine Verbindung (B) SB-271046 ist, und wobei das Medikament optional auch mindestens ein pharmazeutisches Adjuvans umfasst.

2. Medikament nach Anspruch 1 zur Behandlung von Störungen des peripheren Nervensystems oder Störungen des zentralen Nervensystems, besonders von Störungen des zentralen Nervensystems.

3. Medikament nach Anspruch 1 oder 2 zur Behandlung von kognitiven Störungen, Gedächtnisstörungen, Prozessen der senilen Demenz, wie zum Beispiel Alzheimer-, Parkinson- und/oder Huntington-Krankheit, Aufmerksamkeitsdefizitsyndrom, wie zum Beispiel kindliche Hyperkinesie (ADHD, Aufmerksamkeitsdefizit- / Hyperaktivitätsstörung), Epilepsie, Angst, Panik, Depression, Psychose, Schmerz, Schizophrenie, oder zur Verbesserung / Verstärkung der Kognition.

4. Medikament nach einem der Ansprüche 1 bis 3 zur Behandlung von kognitiven Störungen, degenerativen Störungen, Gedächtnisstörungen, ADHD (Aufmerksamkeitsdefizit- / Hyperaktivitätsstörung), Alzheimer-Krankheit, Prozess der senilen Demenz, Lernbehinderungen, die durch degenerative Störungen verursacht sind, Gedächtnis- oder kognitive Funktionsstörung, wie zum Beispiel leichte kognitive Störung, altersbezogener kognitiver Verfall, zerebrale Senilität, vaskuläre Demenz, mit AIDS verbundene Demenz, durch elektrische Schläge ausgelöste Amnesie, Gedächtnisstörung, die mit Depression oder Angstzuständen verbunden ist, kognitive Defekte bei der Parkinson-Krankheit, Down-Syndrom, Schlaganfall, traumatische Hirnverletzung, Huntington-Krankheit und Aufmerksamkeitsdefizitstörung, kindliche Hyperkinesie (ADHD, Aufmerksamkeitsdefizit- / Hyperaktivitätsstörung); besonders ADHD oder für die Verbesserung / Stärkung der Kognition.

## Revendications

1. Médicament contenant une combinaison de substances actives comprenant au moins un composé (A), l'au moins un composé (A) étant du N-[3-(2-diméthylamino-éthyle)-1*H*-indol-5-yl]-6-chloroimidazo[2,1-b]thiazol-5-sulpho-namide et au moins un composé (B), l'au moins un composé (B) étant du SB-271046 et le médicament contenant aussi en option au moins un adjuvant pharmaceutique.

2. Médicament selon la revendication 1 pour le traitement des troubles du système nerveux périphérique ou des troubles du système nerveux central, spécialement des troubles du système nerveux central.

3. Médicament selon la revendication 1 ou 2 pour le traitement des troubles cognitifs, des troubles de mémoire, des processus de démence sénile comme les maladies d'Alzheimer, de Parkinson et/ou de Huntington, des troubles déficitaires de l'attention comme l'hyperkinésie infantile (TDHA, troubles déficitaires de l'attention / hyperactivité), de l'épilepsie, de l'anxiété, de l'angoisse, de la dépression, de la psychose, de la douleur, de la schizophrénie ; ou pour l'amélioration/le renforcement de la cognition.

4. Médicament selon l'une quelconque des revendications 1 à 3 pour le traitement des troubles cognitifs, des troubles dégénératifs, des troubles de mémoire, des TDAH (troubles déficitaires de l'attention / hyperactivité), de la maladie d'Alzheimer, des processus de démence sénile, des inaptitudes à l'apprentissage dues à des troubles dégénératifs, des inaptitudes à l'apprentissage dues à des troubles non dégénératifs, des dysfonctionnements de la mémoire ou de la cognition comme les perturbations cognitives modérées, le déclin cognitif lié à l'âge, la sénilité cérébrale, la démence vasculaire, la démence associée au SIDA, de l'amnésie induite par un choc électrique, les perturbations de la mémoire associées à la dépression ou à l'anxiété, les défaillances cognitives liées à la maladie de Parkinson, au syndrome de Down, aux chocs, aux lésions cérébrales traumatiques, à la maladie d'Huntington et les troubles déficitaires de l'attention, l'hyperkinésie infantile (TDHA, troubles déficitaires de l'attention / hyperactivité) ; spécialement les TDHA, ou pour l'amélioration ou le renforcement de la cognition.
